Europäisches Patentamt

European Patent Office

Office européen des brevets

(11) Publication number: **0 260 829**
**A2**

# EUROPEAN PATENT APPLICATION

(21) Application number: 87307550.1

(22) Date of filing: 26.08.87

(51) Int. Cl.4: **C12P 21/00** , C12N 15/00 ,
G01N 33/577

The applicant has filed a statement in accordance with Rule 28 (4) EPC (issue of a sample only to an expert). Accession number(s) of the deposit(s): ATCC HB-9197, ATCC. HB-9198.

(30) Priority: **15.09.86 US 907016**

(43) Date of publication of application:
**23.03.88 Bulletin 88/12**

(84) Designated Contracting States:
**BE CH DE ES FR GB IT LI LU NL SE**

(71) Applicant: **WESTINGHOUSE ELECTRIC CORPORATION**
**Westinghouse Building Gateway Center**
**Pittsburgh Pennsylvania 15222(US)**

(72) Inventor: **Hunter, Kenneth W.**
**8600 Fox Run**
**Potomac Maryland 20854(US)**

(74) Representative: **Marchant, James Ian et al**
**Elkington and Fife High Holborn House 52/54**
**High Holborn**
**London WC1V 6SH(GB)**

(54) **Antibodies reactive with chlorinated phenols and methods of preparing and using same.**

(57) Monoclonal antibodies that react with chlorinated phenols, particularly, pentachlorophenol, and hybridomas that produce such antibodies are disclosed. Also a method of producing such antibodies by producing an immunogenic conjugate of a chlorinated phenol and a macromolecule carrier, immunizing an animal with the conjugate, obtaining antibody-producing cells from the animal, fusing the cells with tumor cells to produce hybridomas, selecting from among the hydridomas at least one that produces antibodies reactive with the chlorinated phenol, and recovering the antibodies. In addition, polyclonal antibodies to chlorinated phenols and methods of making such antibodies. Also analytic, diagnostic, investigational, separatory, and other methods of using the antibodies, and compositions containing the antibodies for such uses.

EP 0 260 829 A2

# ANTIBODIES REACTIVE WITH CHLORINATED PHENOLS AND METHODS OF PREPARING AND USING SAME

This invention relates to monoclonal and polyclonal antibodies that react with chlorinated phenols and particularly to monoclonal antibodies that react with the toxic chemical pentachlorphenol. The invention is directed to the antibodies, processes of preparing the antibodies, analytic, diagnostic, investigational, separatory, and other methods of using the antibodies, and compositions containing the antibodies for such uses. In addition, the invention is directed to hybridomas that produce the monoclonal antibodies and to methods of making the hybridomas. Finally, the invention is directed to immunogenic conjugates of chlorinated phenols and a macromolecule carrier and to methods of making such conjugates.

The chlorinated phenols are a class of widely used chemicals and are widespread environmental pollutants. One of the most important of the chlorinated phenols is pentachlorophenol (PCP), which is widely used throughout the United States as a pesticide and a preservative. As a pesticide, it is used as an insecticide, fungicide, herbicide, algacide, and disinfectant. PCP finds applications in a wide variety of industries, including those manufacturing wood and wood products, construction materials, leather, paper, and rope. It is also used in water systems to control algae and fungi. The large majority of PCP is used in the treatment of wood products, primarily as a fungicide and bacteriocide. In construction materials, it may be found in tiles, shingles, concrete, asbestos, pressboard, wallboard, and insulation. It is used in leather to prevent deterioration during tanning. It is used as a preservative in paint. The textile industry uses it to prevent processed materials from developing mildew. The chemical may also come in contact with food because of its applications in paper, adhesives, and plastics, which are used in packaging.

In view of its uses and physical characteristics, which enhance its dispersal in the environment, PCP is a major pollutant. It has been found in house dust, air, and water. As a free phenol, it is readily adsorbed on many surfaces, particularly soil. The sodium salt is readily adsorbed by suspended particulate matter in water.

PCP is highly toxic for mammalian species, including humans. It is included on the Environmental Protection Agency's list of priority pollutants. Thus, it is important to be able to detect the presence of PCP and other toxic chlorinated phenols in water, soil, food, and other materials and then to quantify the amount present.

Analysis of PCP, particularly in water, is difficult and costly. The present EPA-approved method of analysis is gas chromatography using electron capture and/or flame ionization detectors. See 49 Fed. Reg. 58-66 (October 26, 1984). This method is costly, time consuming, and labor intensive. The equipment is complex and expensive, and it requires highly trained operators. The method involves an extensive extraction and cleanup procedure for each sample. Thus, it usually takes more than a day to analyze a sample at a cost of approximately $100.

Immunoassays of various types are used for the detection of small concentrations of compounds. They involve the use of antibodies to the substance sought to be detected and are generally fairly sensitive, specific, reasonably cost-effective, and easy to use. However, such immunoassays have not been used for the detection and quantification of chlorinated phenols. In fact, antibodies to PCP and other chlorinated phenols have not been produced prior to this invention.

The preparation was undertaken of novel antibodies reactive in any way with chlorinated phenols, particularly pentachlorophenol, since such antibodies, if, indeed, they could be produced, would satisfy a number of critical needs not fulfilled by existing analytical technology. Such antibodies, would bind to pentachlorophenol, thus allowing the detection of this toxic chemical by a variety of rapid, simple, and cost effective immunoassay techniques. They would be particularly useful for analyzing PCP in water, since the PCP would not have to be extracted first.

Further, there was undertaken the preparation of novel, self-reproducing cell lines that produced monoclonal antibodies to chlorinated phenols, particularly pentachlorophenol. In contrast to polyclonal antibodies, monoclonal antibodies are derived from a single B lymphocyte clone, which makes them very specific and homogeneous. In 1975, Kohler and Milstein, Nature, 265:495 (1975), which is incorporated herein by reference, first described how monoclonal antibodies directed to sheep red blood cells may be prepared by fusing a specific antibody-producing B lymphocyte with a tumor cell, resulting in an "immortal" self-reproducing hybrid clone (or "hybridoma") that can synthesize, in a test tube (in vitro) or an animal (in vivo), a single, monoclonal antibody. Such a hybridoma is, in effect, a self-reproducing cell "factory" which can produce a potentially limitless supply of an antibody with single, pre-defined specificity.

Thus, such antibodies would be more useful than polyclonal antibodies as detection reagents because of their exquisite specificity. Likewise, this specificity would also render such monoclonal antibodies more useful for immunological and biochemical studies of PCP, for affinity purification of PCP, and for the neutralization and/or removal of PCP from water or other PCP-containing materials. Furthermore, unlike conventional polyclonal antibodies, monoclonal antibodies reactive with PCP could be produced in a potentially limitless and homogeneous supply, thus avoiding the problems imposed by the low immunogenicity of PCP/protein conjugates, the variability of polyclonal antibody response, and the resulting limitations in achievable levels or supplies of anti-PCP antibodies available for environmental and other applications.

Accordingly, the present invention resides in a monoclonal antibody reactive with a chlorinated phenol and a hybridoma which produces such an antibody. Preferably, the monoclonal antibody reacts with pentachlorophenol.

The invention further includes a method of producing monoclonal antibodies reactive with a chlorinated phenol by producing an immunogenic conjugate of the chlorinated phenol and a macromolecule carrier, immunizing an animal with the conjugate, obtaining antibody-producing cells from the animal, fusing the cells with tumor cells to produce hybridomas, selecting from among the hybridomas at least one that produces antibodies reactive with the chlorinated phenol, and recovering the produced antibodies from the selected hybyridoma.

The present invention also provides an in vivo method of producing monoclonal antibodies reactive with a chlorinated phenol by intraperitoneally placing in a histocompatible or immunosuppressed host a hybridoma that produces such antibodies and recovering the produced antibodies from the ascites fluid of the host.

The present invention also provides a method of producing a continuous cell line that produces monoclonal antibodies to a chlorinated phenol by producing an immunogenic conjugate of the chlorinated phenol and a macromolecule carrier, immunizing an animal with the conjugate, obtaining antibody-producing cells from the animal, fusing the antibody-producing cells with tumor cells to produce hybridomas, selecting from among the hybridomas a hybridoma that produces antibodies reactive with the chlorinated phenol, and clonally expanding the selected hybridoma into a cell line.

The invention also provides a method for detecting the presence or concentration of a chlorinated phenol in a sample by adding monoclonal antibodies reactive with the chlorinated phenol to the sample and determining the presence or concentration of the chlorinated phenol by an immunoassay, wherein the monoclonal antibodies are used as a reagent in the immunoassay.

The invention also provides a composition for determining the presence or concentration of a chlorinated phenol in a sample, which composition comprises an effective amount of a monoclonal antibody reactive with the chlorinated phenol in an acceptable carrier.

The invention also provides an immunological procedure for the isolation or removal of a chlorinated phenol from a mixture on the basis of a selective immunological reaction with an antibody specific for the chlorinated phenol in which a monoclonal antibody reactive with the chlorinated phenol is used as the antibody.

The invention also provides a composition for isolating or removing a chlorinated phenol from a mixture containing it, which comprises an effective amount of a monoclonal antibody reactive with the chlorinated phenol immobilized on an acceptable matrix or in admixture with an acceptable carrier.

The invention further provides polyclonal antibodies reactive with a chlorinated phenol and a method for producing such antibodies by producing an immunogenic conjugate of the chlorinated phenol and a macromolecule carrier, immunizing an animal with the conjugate, removing blood from the animal, separating the serum from the blood, and recovering the antibodies from the serum.

The invention further provides an immunogenic conjugate comprising a chlorinated phenol covalently bonded to a macromolecule carrier and a method for producing such a conjugate by covalently attaching a chemical linker to the macromolecule carrier and covalently attaching a derivative of the chlorinated phenol, such derivative containing a reactive group, to the chemical linker, thereby forming the immunogenic conjugate.

The present invention thus relates to antibodies reactive with chlorinated phenols. As used herein, the term "chlorinated phenol" means a phenol with a chlorine atom attached at any one or more of positions 2-6 of the benzene ring.

In one embodiment, the antibodies are polyclonal antibodies reactive with a chlorinated phenol. In another and preferred embodiment, the antibodies are monoclonal antibodies that are reactive with a chlorinated phenol, preferably pentachlorophenol.

3

In a particularly preferred embodiment, the monoclonal antibodies of the present invention have the characteristics of the monoclonal antibodies produced by the hybridoma cell lines ATCC HB 9197 and ATCC HB 9198 or mutants or variants thereof. ATCC HB 9197 and ATCC HB 9198 are biologically pure cultures available from the permanent collection of the American Type Culture Collection (ATCC), 12301 Parklawn Drive, Rockville, Maryland USA 20852 and were deposited on September 12, 1986. ATCC HB 9198 was produced by fusion of a rat B lymphocyte and a rat myeloma cell and ATCC HB 9197 was produced by fusion of a mouse B lymphocyte and a mouse plasmacytoma cell. The immunoglobulins (antibodies) produced by these hybridomas are of the IgG glass. The monoclonality of the ATCC HB 9197 and ATCC HB 9198 antibodies was insured by re-cloning the hybridomas which produced them.

The present invention also includes hybridomas and continuous cell lines that produce monoclonal antibodies to chlorinated phenols. Preferably, those hybridomas and cell lines produce monoclonal antibodies to PCP. Most preferably, the continuous cell lines of the present invention have the characteristics of the hybridoma cell lines having ATCC Accession Numbers HB 9197 and HB 9198 or mutants or variants of those cell lines. The invention also encompasses individual cells of that cell line or any continuous cell line that produces the monoclonal antibodies of the present invention.

Persons skilled in the art can use known techniques to produce mutants or variants of ATCC HB 9197 or ATCC HB 9198. Such mutants or variants are encompassed within the present invention as long as they produce monoclonal antibodies reactive with PCP. In addition, persons skilled in the art, using techniques known in the art and the teachings disclosed herein, will be able to produce monoclonal antibodies reactive with PCP and hybridomas and cell lines that produce such monoclonal antibodies, which will have different characteristics from ATCC HB 9197 or ATCC HB 9198 or the antibodies produced by such cell lines. Nevertheless, such monoclonal antibodies and the hybridomas or cell lines producing them are within the scope of the present invention. The monoclonal antibodies produced by ATCC HB 9197 or ATCC HB 9198 will prevent other monoclonal antibodies that would otherwise react with PCP, which have been produced by other hybridomas, from so reacting with PCP. Such other monoclonal antibodies are encompassed within the scope of the present invention.

The hybridomas and continuous cell lines of the present invention may be produced by:

- (a) producing an immunogenic conjugate of a macromolecule carrier and the particular chlorinated phenol to which a monoclonal antibody is sought;
(b) immunizing an animal with the conjugate;
(c) obtaining antibody-producing cells from the animal;
(d) fusing the antibody-producing cells with tumor cells to produce hybridomas;
(e) selecting from among the hybridomas a hybridoma that produces antibodies reactive with the chlorinated phenol. The selected hybridoma or hybridomas may then be cloned to produce a cell line.

The monoclonal antibodies of the present invention may be recovered from the selected hybridoma or hybridomas before or after they are expanded by cloning into a continuous cell line. As previously mentioned, in a preferred embodiment of the invention, the chlorinated phenol is PCP. Thus, the preferred monoclonal antibodies are monoclonal antibodies to pentachlorophenol.

In an alternative embodiment, the hybridomas of the present invention may be produced by the fusion of a tumor cell with any cell that produces antibodies to a chlorinated phenol. The hybridomas produced by this embodiment can be used to produce the monoclonal antibodies of the invention by culturing them in a suitable medium and recovering the antibodies from the medium.

Substances with molecular weights of approximately 1000 or less, such as pentachlorophenol (molecular weight 266), do not ordinarily induce the production of antibodies; i.e., they are non-immunogenic. However, such substances often can be chemically attached to larger, immunogenic carrier molecules, such as a carbohydrate or a protein, in order to induce the production of antibodies against the smaller substance, which is known as a hapten. The general techniques for producing an immunogenic conjugate of a hapten and a macromolecule carrier are known in the art. For example, see U.S. Patent No. 4,456,691 to Stark, issued June 26, 1984, and Albro et al., Toxicol Appl. Pharmacol. 50, 137-146 (1979).

The method of Albro et al. or Stark involves covalently bonding the hapten to the macromolecule carrier through a chemical bridge or linker. The chemical linker is a bifunctional molecule that contains reactive groups on opposite ends. These reactive groups allow the chemical linker to react with reactive groups on the macromolecule and on the hapten so that one end of the chemical linker is covalently bonded to the macromolecule and the other end of the chemical linker is covalently bonded to the hapten. In the method of Albro et al. or Stark, the chemical linker is added first to the hapten and the resulting compound is then added to the macromolecule carrier through the reactive group at the other end of the chemical linker.

It is known by those skilled in the art that antibodies raised against small chemical compounds conjugated to carrier proteins tend to recognize not only the structure of the chemical, but also the structures of the linker between chemical and protein, and even structures on the protein adjacent to the site of attachment of the linker. The result of such recognition of ancillary structures is the inability, or markedly reduced ability, of the antibody to bind to the free chemical. This is particularly a problem for monoclonal antibodies. This problem must be overcome if monoclonal antibodies to chemicals are to be useful for quantitative analysis. With appropriate conjugation and selection methods, monoclonal antibodies that bind to the free or unbound substance can be prepared and identified.

The present invention does not use the general method of Albro et al. or Stark to attach a chlorinated phenol to a macromolecule carrier. In the case of a chlorinated phenol, the hydroxyl group must be preserved so that it is available to react with the antibodies sought to be made. Since the chlorine groups are unreactive, a derivative containing a reactive group in any one of the 2-6 positions of the benzene ring must be used. However, applying the method of Stark or Albro et al. results in polymerization and precipitation of the starting chlorinated phenol.

Therefore, in the method of the present invention, the chemical linker is covalently attached first to the macromolecule carrier and the derivative of the chlorinated phenol is then attached to the other end of the chemical linker, thereby forming the immunogenic conjugate. Preferably, the reactive group is in the meta or para position with respect to the hydroxyl group on the chlorinated phenol derivative. Thus, when the chemical linker/protein moiety is attached to the chlorinated phenol to form the conjugate, the hydroxyl group is easily available for reacting with antibodies. Various chemical linkers may be used, depending upon the length of the linkage desired between the chlorinated phenol and the macromolecule. The choice of such a linker will be apparent to those of ordinary skill in the art in view of the teachings contained herein. Preferable reactive groups on the chemical linker, the chlorinated phenol derivative, and the macromolecule carrier include amino, hydroxyl, and carboxyl groups.

Once the immunogenic conjugate has been produced, it is used to immunize an animal host by techniques known in the art. Such techniques usually involve inoculation, but they may involve other modes of administration. A sufficient amount of the conjugate is administered to create an immunogenic response in the animal host.

Any host that produces antibodies to the conjugate may be used. Conventionally used animals include rabbits and rodents, such as rats or mice. Mice and rats are preferred for the present invention.

Once the animal has been immunized and sufficient time has passed for it to begin producing antibodies to the conjugate, polyclonal antibodies may be recovered by techniques known in the art. The general method comprises removing blood from the animal and separating the serum from the blood. The serum, which contains antibodies to the chlorinated phenol used as the hapten in the preparation of the conjugate, may be used as an antiserum to the chlorinated phenol. Alternatively, the antibodies can be recovered from the serum. Affinity purification is a preferred technique for recovering purified polyclonal antibodies to a chlorinated phenol from the serum.

In the embodiment of this invention relating to monoclonal antibodies, antibody-producing cells are recovered from the immunized animal. Although any antibody-producing cells may be used, B lymphocytes obtained from the animal's spleen are preferred.

The antibody-producing cells are fused with tumor cells to produce hybridomas. As used herein, the term "tumor cell" includes any cell that is capable of fusing with an antibody-producing cell to create a hybrid "immortal" cell; i.e., one which is capable of continuous growth in vitro. Preferred tumor cells are antibody-producing cells which have been transformed and which have lost their ability to produce immunoglobulin. Such cells include rat myeloma cells and mouse plasmacytoma cells. Particularly preferred are mouse plasmacytoma cells or rat myeloma cells that are deficient in the enzyme hypoxanthine-quanine phosphoribosyl transferase (HGPRT), which allows the selection of hybridomas from unfused antibody-producing cells or plasmacytoma or myeloma cells when grown on a medium containing hypoxanthine, aminopterin, and thymidine.

Various tumor cells useful for fusing with antibody-producing cells are known and readily available to those skilled in the art. One such type of cell is the mouse plasmacytoma cell line P3-X63-Ag8.653, described in Kearney, J. Immunology, 123, 1548 (1979). Another cell line is the rat myeloma cell line YB2/0, described in Milstein, J. Cell Biology, 93:576-582 (1982). These cell lines are available from American Type Culture Collection, Rockville, Maryland, where they are designated ATCC CRL 1580 and ATCC CRL 1662, respectively.

It should be noted that the antibody-producing cell and the tumor cell can be from different animal species. For example, see Nowinski et al., Science, 210:537 (1980).

As mentioned previously, once the cells have been fused, it is necessary to separate the hybridomas from the unfused cells. The antibody-producing cells will normally die after a few days in culture, but the tumor cells are "immortal". However, by using tumor cells that are deficient in HGPRT and growing the fused cells on a medium containing hypoxanthine, aminopterin, and thymidine, the hybridomas will be naturally selected for, since the tumor cells are unable to survive on such a medium. However, other known selection techniques may also be used.

After the hybridomas are selected, they are evaluated to determine which are producing antibodies to the chlorinated phenol. Various immunoassays known to those skilled in the art may be used to evaluate the culture supernatants of the hybridomas. Care must be taken to identify hybridomas that are producing monoclonal antibodies only to the chlorinated phenol rather than to the phenol-macromolecule carrier. That is, the monoclonal antibodies which are desired, useful, and provided by this invention are those that react with free, i.e., unconjugated or unbound, chlorinated phenols.

The preferred selection technique is an initial screen by an enzyme immunoassay (EIA) to identify hybridomas producing antibodies to the conjugate or to the chlorinated phenol. These hybridomas are then screened by a competitive inhibition enzyme immunoassay (CIEIA) that evaluates the ability of a free chlorinated phenol, such as PCP, to inhibit the binding of monoclonal antibodies to the phenol/protein carrier. The CIEIA is conducted according to the method disclosed in Hunter et al., FEBS Lett. 149:147-151 (1982).

Once the monoclonal antibody-producing hybridomas have been selected, the antibodies can be recovered from such hybridomas by known techniques. Generally, it is useful to clone one or more of the monoclonal antibody-producing hybridomas to expand it into a continuous cell line that can be used to produce the monoclonal antibodies in quantity.

The previously mentioned methods of producing the monoclonal antibodies of the present invention are in vitro methods. The present invention also comprises an in vivo process for producing monoclonal antibodies to chlorinated phenols. Such antibodies are produced by placing a hybridoma that produces the antibodies intraperitoneally in a histocompatible or immunosuppressed host. This causes the host to produce ascites tumors which, in turn, produce a fluid that contains the monoclonal antibodies produced by the hybridoma. After a sufficient time has passed for the antibodies to have been produced in sufficient quantities, they may be recovered by known techniques. For example, the ascites fluid may be removed and the monoclonal antibodies recovered in pure form by affinity purification. This method is particularly suitable for producing the monoclonal antibodies in commercially useful quantities.

Just as a variety of different systems and methods might be employed to produce monoclonal antibodies reactive with chlorinated phenols, so do a variety of monoclonal antibodies result from these measures that are distinct from the antibody illustrated in the Examples below. However, such monoclonal antibodies, whose production is enabled by the teachings therein, are still clearly within the scope of this invention. The salient feature of such antibodies, for the purposes of this invention, besides their monoclonality, is their reactivity in any way with chlorinated phenols, regardless of the species of origin, isotype, molecular specificity, affinity, method of production, or particular type of hybridoma employed in their production.

The monoclonal and polyclonal antibodies of the present invention may be used to identify chlorinated phenols, particularly PCP, in materials and to determine the concentration of the chemical in those materials. Such materials include, for example, soil, water, food, and body fluids. When used as a reagent in various immunoassays for determining the presence or concentration of a chlorinated phenol, the antibodies of the present invention provide an improved assay. Detection is more convenient, rapid, sensitive, and specific. The immunoassays in which the antibodies of the present invention may be used include, but are not limited to, radioimmunoassay, competition immunoprecipitation assay, enzyme-linked immunoabsorbent assay, and immunofluorescence assay. The monoclonal antibodies of the present invention are generally the preferred antibodies, although the polyclonal antibodies are preferred in certain applications.

A composition for determining the presence or concentration of a chlorinated phenol in material in accordance with the present invention contains a concentration of antibodies to the chemical effective to detect the presence of the chemical or to quantify its amount. The antibodies can be mixed with or attached to any suitable carrier, such as a latex particle or plastic microtiter plate. They may also be conjugated with an enzyme or dye or radio-labelled, depending upon what immunological method is employed. Consequently, any assay system which employs monoclonal or polyclonal antibodies reactive with chlorinated phenols, including pentachlorophenol, is embraced by this invention.

The monoclonal and polyclonal antibodies of this invention are also useful for the isolation, purification, neutralization, and/or removal of chlorinated phenols from complex mixtures of solutions on the basis of a selective immunological reaction. The use of antibodies reactive with a chlorinated phenol represents an improvement over the known methods.

The attributes of the monoclonal antibodies of the invention make them particularly useful for these applications, compared to polyclonal antibodies. These attributes include their great specificity and their availability in virtually limitless quantities, which permits their use on a large, industrial or commercial scale. For example, monoclonal antibodies to PCP are useful to separate and purify PCP from a mixture of other chlorinated phenols or similar organic compounds. The mixture is brought into contact with immobilized monoclonal antibodies to PCP, which separates the PCP from the mixture by forming immobilized complexes of the PCP bound to the antibody. After the mixture is removed, the PCP is separated from the antibodies and recovered in purified form by known techniques.

A composition in accordance with the invention useful for purifying or removing a chlorinated phenol from complex mixtures contains an effective amount of the monoclonal antibody of this invention, immobilized on an acceptable matrix or in admixture with an acceptable carrier, to permit reaction and binding with the chlorinated phenol. However, for certain mixtures, polyclonal antibodies may be preferred.

The monoclonal antibodies of this invention are also useful reagents for research related to the structure and function of chlorinated phenols, particularly pentachlorophenol. Their exquisite specificity allows them to be used for immunochemical and structure-activity analyses of these chemicals, and makes them more useful in these applications than less specific polyclonal antibodies.

A composition in accordance with the present invention useful as an investigational reagent contains an amount of monoclonal antibody effective in providing information upon mixture with a chlorinated phenol and subsequent analysis. Determination of the amount of antibody necessary to accomplish a particular research goal depends upon the specific types of investigation involved, and it is readily within the skill of one carrying out such research.

The invention will now be illustrated with reference to the following Examples:

## Example 1

### Preparation of Tetrachlorohydroquinone-Protein Conjugates Used in Raising an Immune Response to PCP

In this procedure, protein was first treated with succinic anhydride to introduce free carboxylic acid groups which can be esterified to one of the hydroxyls of tetrachlorohydroquinone. This produces an adduct that presents a structure similar to PCP to the rat immune system.

### Protein succinylation

Since Tetrachlorohydroquinone can react through both the ortho and para positions, attempts to succinylate this compound for subsequent conjugation to protein resulted in polymerization and precipitation of the starting compound. To avoid this problem, the protein was succinylated, and upon reaction with tetrachlorohydroquinone, yielded a substantial amount of tetrachlorohydroquinone-protein conjugate, with less precipitation of the starting compound. This was done in the following manner.

45.2 mg (0.001 m moles) of bovine serum albumin (BSA) was dissolved in 3 ml of deionized water. 420 mg (4.2 m moles) of succinic anhydride was added in small increments with constant stirring at room temperature. The pH was monitored and maintained between 7 and 8 by the addition of 1.0 N sodium hydroxide solution.

After the last of the succinic anhydride was added and the pH stabilized, the reaction was allowed to continue for 30 min. At this point the pH was adjusted to 2.5 with 1.0 N hydrochloric acid and the protein solution was dialysed against distilled water overnight. The dialysed protein was lyophilized. Porcine thyroglobulin (Thy) and goat immunoglobulin were also successfully treated in this manner.

Esterification of Tetrachlorohydroquinone to the Free Succinate Carboxyl Groups

40 mg of succinylated bovine serum albumin (approx. $6.2 \times 10^{-7}$ moles) were dissolved in 15 ml of dry DMSO. While stirring at room temperature, 12.4 mg ($5.0 \times 10^{-5}$ moles) tetrachlorohydroquinone, 150 mg ($7.8 \times 10^{-4}$) 3-dimethyl-aminopropyl carbodiimide and 1 mg dimethylaminopyridine were added to the protein solution. The reaction proceeded for 4 hours, then the mixture was placed into dialysis against $2 \times 4$ liters of distilled water at 4°C for 18 hours. The dialysed protein solution was lyophilized. The yield was 38 mg of BSA conjugate. Porcine thyroglobulin was also treated successfully by this procedure.

Epitope Density Determinations

Epitope densities were determined spectrophotometrically by using the difference in optical densities between equal concentrations of derivatized protein and underivatized protein in 0.1 N NaOH at 270.4 nm. Using the extinction coefficient of tetrachlorohydroquinone at that wavelength, the molar ratios of protein to hydroquinone residues for thyroglobulin and bovine serum albumin were found to be 123 and 55, respectively.

Example 2

Preparation of Alternative Conjugate

To improve the sensitivity of the immunoassay for PCP, a conjugate was prepared that presented a weakly cross-reacting congener of PCP. By lowering the affinity (binding strength) of the conjugate-anti-PCP interaction, free PCP at lower concentrations could compete more effectively. This procedure therefore increased the sensitivity of the competitive inhibition enzyme immunoassay for PCP.

4-(4-hydroxy-3,5-dichlorophenyl)butyric acid was prepared according to the method of Martin, J. Am. Chem. Soc., 38, 1438 (1936). 180 mg (1 m mole) of 4-(4-hydroxyphenyl)butyric acid was stirred in 1.5 ml of sulfuryl chloride at room temperature for 1 hr. The precipitate formed was filtered to give 35 mg of 4-(4-hydroxy-3, 5-dichlorophenyl)butyric acid; H NMR (DMSO-$d_6$) ∅ 1.73 (2H, broad quintet, J = 7 Hz, -CH$_2$-), 2.17 (2H, triplet, J = 6 Hz, ≥C-CH2-), 2.50 (2H, -CH$_2$-CO), 5.80 (1H, broad, exchange with D$_2$O, -CO$_2$H), 7.10 (2H, singlet, aromatic H).

To 7 mg (0.028 m mole) of 4-(4-hydroxy-3,5-dichlorophenyl) butyric acid dissolved in 2 ml of dioxane was added 24 microliters (0.1 m mole) of tri-n-butylamine and 13 microliters (0.1 m mole) of isobutyl chloroformate sequentially. The solution was stirred at room temperature for 0.5 hr and then 1 ml was added dropwise to a stirred, ice-chilled solution of 100 mg of BSA in 0.1 ml of 1 N sodium hydroxide, 2 ml of water, and 2 ml of dioxane. The resulting mixture was stirred 1 hr in ice bath and 1 hr at room temperature. The resulting 4-hydroxy-2,3,5,6-tetrachlorophenyl succinate-BSA was dialyzed in deionized water overnight and then freeze-dried.

Example 3

Production of Hybridomas

Six week old female Sprague-Dawley rats were injected intramuscularly with 500 micrograms of 4-hydroxy-2,3,5,6-tetrachlorophenyl succinate-BSA emulsified in complete Freund's adjuvant. After three weeks, the mice were boosted with 50 micrograms of the same conjugate in 0.15 M NaCl. The donor rats were killed by cervical dislocation three days following the last immunization; the spleen was removed aseptically and placed in a 35 mm plastic Petri dish with 5 ml of cold Dulbecco's Minimal Essential Medium (DMEM). The spleen was disassociated into a single cell suspension, and the cells were washed twice with cold DMEM and resuspended in the same medium.

An immunoglobulin non-secreting rat myeloma cell line (YB2/0) deficient in the enzyme hypoxanthine-guanine phosphoribosyl transferase (HGPRT-, EC 2.4.2.8), as disclosed by Milstein, Journal of Cell Biology , 93:576-582 (1982), was used as the fusion partner. This cell line is available from the American Type Culture Collection, Rockville, Maryland, where it is designated ATCC CRL 1662. The myeloma cell line was maintained in DMEM containing 10% fetal bovine serum and further supplemented with 2 mM L-glutamine, 1% sodium pyruvate, 1% non-essential amino acids, 100 IU/ml penicillin, and 100 micrograms per milliliter streptomycin. For three days prior to fusion, 0.1 mM 8-azaguanine was added to the myeloma cells in order to kill any HGPRT+ revertants. On the day of fusion, the myeloma cells were harvested from 75 cm² culture flasks, washed once, and resuspended in serum-free DMEM. The myeloma and previously harvested spleen cells were counted and their viability assessed by Trypan blue dye exclusion.

The fusion technique used was modified from that of Gefter et al., Somatic Cell Genetics, 3:231, 1977. Described below is the fusion experiment which yielded ATCC HB 9198; other fusion experiments performed in an identical manner produced other rat hybridoma clones.

Mouse hybridoma clones, such as ATCC HB 9197, were prepared similarly by utilizing an immunoglobulin non-secreting mouse plasmacytoma cell line (P3-X63-Ag8.G53) deficient in HGPRT, as disclosed by Kearney, Journal of Immunology, 123:1548, 1979, as the fusion partner. This cell line is available from the American Type Culture Collection, Rockville, Maryland, where it is designated ATCC CRL 1580.

To a sterile 50 ml conical plastic tube was added $1.0 \times 10^8$ spleen cells and $1.0 \times 10^7$ myeloma cells. The myeloma-spleen cell suspension was centrifuged at $250 \times g$ for 10 minutes at room temperature and the medium was decanted to near dryness. The cell pellet was loosened by gently flicking the tube, and 1 ml of 50% polyethylene glycol (MW 1400) in DMEM, without serum, was added in drops over 45 seconds. The tube was gently agitated during this process. One minute later, another 2 ml of DMEM was added over 2 minutes. An additional 20 ml of DMEM was added over the next 2 minutes, and the cells were pelleted by centrifugation at $250 \times g$ for 10 minutes at room temperature.

The fluid was decanted, and 40 ml of enriched selection medium was added. This medium was composed of DMEM containing 10% fetal bovine serum and supplemented with 2 mM L-glutamine, 1% sodium pyruvate, 1% non-essential amino acids, 100 IU/ml penicillin, 100 micrograms per milliliter streptomycin, 1 mM oxaloacetic acid, 10% NCTC 109, and 0.2 micrograms per milliliter bovine pancreatic insulin. The medium also contained $1.0 \times 10^{-4}$ M hypoxanthine, $4.0 \times 10^{-7}$ M aminopterin, and $1.6 \times 10\text{-}5$ M thymidine (HAT). Aminopterin is toxic for cells that lack the enzyme HGPRT and therefore kills all unfused myeloma cells. Fused cells (hybridomas) survive in HAT because they obtain HGPRT from the B lymphocyte (spleen cell) fusion partner.

## Example 4

### Selection of Hybridomas Producing Monoclonal Antibodies to PCP

Aliquots of 0.2 ml containing $5.0 \times 10^5$ cells were transferred from the mixture produced in Example 3 into individual wells of several sterile flat-bottomed microtiter plates. The plates were incubated at 37°C in a humidified atmosphere consisting of 6% $CO_2$ and 94% air. Fresh selection medium was added on alternate days for the next 14 days.

On day 14, the microculture supernatants were tested for antibodies reactive with PCP by the following EIA. Purified 4-hydroxy-2,3,5,6-tetrachlorophenyl succinate-Thy conjugate was dissolved at a concentration of 10 micrograms per milliliter in coating buffer (phosphate buffered saline (PBS), pH 7.4) and dispensed in 50 microliter aliquots into 96-well polyvinyl chloride microtiter plates (Dynatech Laboratories, Inc., Chantilly, VA). After overnight incubation at 4°C, the conjugate solution was removed and the wells were washed five times with PBS-Tween (0.5 ml/1 Tween-20). 50 microliter test samples were added to the wells, the plates were incubated at 4°C for 30 minutes, and then were washed five times with PBS-Tween. The final two steps, separated by PBS-Tween washes, were as follows: addition of 50 microliters of a 1:500 dilution of goat anti-rat IgG/IgM conjugated with the enzyme alkaline phosphatase (Sigma Chemical Co., St. Louis, MO) followed by incubation at 4°C for 30 minutes, then the addition of 50 microliters of p-nitrophenyl phosphate (Sigma-104) substrate, 1 mg/ml in 10% diethanolamine, pH 9.8, and incubation at 25°C for 30 minutes. Absorbance was read at 405 nm in a Bio-Tek micro-EIA spectrophotometer (Bio-Tek Instruments, Inc., Winooski, VT).

Furthermore, to verify that the antibodies were truly reactive with PCP, and not only with the 4-hydroxy-2,3,5,6-tetrachlorophenyl succinate-Thy conjugate, the culture supernatants were preincubated with $1.0 \times 10^{-3}$ M PCP. This so-called competitive inhibition enzyme immunoassay (CIEIA) resulted in the inhibition of antibody binding to the solid phase bound conjugate by fluid phase PCP. Whereas 16 of 192 cultures were positive by EIA (8%), only 5 were positive by CIEIA (2.6%, showing at least 50% inhibition with $1.0 \times 100^{-3}$ M PCP). Positive cultures were expanded in number and were transferred to 35 cm² flasks. The medium from these cultures was retested, and those hybridomas maintaining antibody secretion were cryopreserved and stored at -179°C in liquid nitrogen vapor phase.

## Example 5

### Cloning of Hybridoma Culture that Produces Monoclonal Antibodies to PCP

One of the positive hybridoma cultures described in Example 4 was selected for cloning by limiting dilution. 100 microliter aliquots containing 0-1 cells were transferred to several hundred individual wells in sterile flat-bottomed microliter plates that had been previously seeded with mouse thymocytes, which serve as "feeder" cells for the hybridomas. After 21 days, the cloned cultures were tested again by EIA and CIEIA for antibodies reactive with PCP, and one positive clone was selected for further study. This cloned hybridoma, which has been deposited in the American Type Culture Collection under accession number ATCC HB 9198, was expanded in numbers, cryopreserved, and stored in the same manner as the parental cells lines from which it was derived.

## Example 6

### In Vitro Production of Monoclonal Antibodies to PCP

Stationary cultures of ATCC HB 9198 were grown in 75 cm² culture flasks containing DMEM supplemented as described in Example 3. After 5-7 days of incubation at 37°C under 6% $CO_2$, 1-2 liters of culture fluid containing approximately 10 micrograms per milliliter of anti-PCP antibody were obtained.

## Example 7

### In Vivo Production of Monoclonal Antibodies to PCP

An in vivo method for obtaining large amounts of monoclonal antibodies involved the adaptation of ATCC HB 9198 to grow as an "ascites" tumor. Female Sprague-Dawley rats were "primed" by intraperitoneal injection of 0.5 ml of pristane (2,6,20,14-tetramethylpentadecane). Pristane is a sterile irritant which elicits a serous secretion ("ascites") in the peritoneal cavity rats, which acts as a growth medium. Approximately 7-10 days following the pristane injection, aliquots containing $5.0 \times 10^6$ actively growing hybridoma cells harvested from in vitro cultures as described in Example 7 were inoculated into the peritoneal cavities of primed rats. Ascites tumors were formed, which grew rapidly in the fluid microenvironment of the rat peritoneal cavity and secreted large quantities (e.g., 2-10 mg/ml) of monoclonal antibodies reactive with PCP. Routinely, 15-20 ml of ascites fluid was removed from each rat by aspiration. The antibody-secreting tumor cells were separated from the antibody-containing fluid phase by centrifugation.

## Example 8

### Affinity Purification of Monoclonal Antibodies to PCP

The purpose of the affinity purification procedure was to remove extraneous proteins from fluids which contain monoclonal antibodies to PCP, and to provide anti-PCP antibodies in pure form.

The following chemicals were used in this procedure: sodium chloride; ethanolamine - HC1; propionic acid; glycine HC1; 4-hydroxy-2,3,5,6-tetrachlorophenyl succinate-Thy conjugate at 5.0 - 7.5 mg/ml in 0.9% NaCl; and gluterdialdehyde - activated silica affinity absorbent.

The following apparatus were used in this procedure: Buchner funnel; rotator; Amicon concentrator (ultrafiltration stirred cell); YM50 and YM300 filters for above (50 kD and 300 kD cutoff filters, respectively); and nitrogen.

To prepare the antibody from ATCC HB 9198 for affinity purification, approximately 600 ml of supernatant from cells secreting monoclonal antibodies to PCP was concentrated to approximately 30 ml under nitrogen pressure through a 300 kD cutoff filter. Both the filtrate and the concentrated supernatant were tested in a CIEIA for antibody activity. The antibody-containing fraction was then concentrated 10-20X by filtration under nitrogen pressure through a 50 kD filter. The concentrate was tested by CIEIA to ensure antibody activity, and the antibody-containing fraction was dialyzed overnight at 4°C against PBS.

To prepare the affinity adsorbent, between 9 and 10 ml of conjugate solution was mixed with 3 grams of gluterdialdehyde-activated silica adsorbent and rotated overnight at 4°C. The column material was then washed with 1.5% NaCl on a Buchner funnel with #1 filter paper until eluate was protein-free (O.D. 280 less than 0.005). The column material was charged by mixing with 0.3 $\underline{M}$ ethanolamine HCl (pH 7.5) for 1 hour on the rotator at room temperature. The column material was then washed, in order, with 0.9% NaCl, 0.5$\underline{M}$ propionic acid, and 0.9% NaCl on a Buchner funnel.

The antibody was coupled to the column material by adding approximately 30 ml of antibody solution to the column material in a 50 ml tube. The tube was rotated overnight at 4°C. The coupled matrix was then poured into a 20 cc syringe with a glass wool plug at the bottom. The column was washed with 3% NaCl until the eluant was protein-free. The antibody was eluted with 0.2 $\underline{M}$ glycine-HCl, pH 2.0, and samples of 3-4 ml were collected and the O.D. 280 determined for each. Those with O.D. greater than 0.2 were pooled. The pH was raised to 7.0 with 1 N NaOH, and then the solution was dialyzed overnight at 4°C against PBS.

## Example 9

## Demonstration of Analytical Utility of Monoclonal Antibodies to PCP

To verify that the antibodies from ATCC HB 9198 interacted with free PCP, and to further demonstrate that such antibodies could be used to quantify PCP levels, a CIEIA was performed. This assay was similar to the straight EIA described in Example 4, except that the antibodies were preincubated for 1 hour with various concentrations of PCP. Interaction of the antibodies with PCP in solution inhibited their binding to conjugate on the solid phase, thereby decreasing the ultimate color reaction. Various concentrations of PCP in PBS-T with 25% methanol were preincubated with 0.9 micrograms/ml of affinity purified anti-PCP antibody (prepared as described in Example 8) for 1 hour at room temperature, then aliquots of the mixtures were transferred to PVC plates coated with 4 micrograms/ml of 4-hydroxy-3,5-dichlorophenyl succinate-BSA. After a 30 minute incubation, the plates were washed and further processed as discussed in Example 4 for EIA.

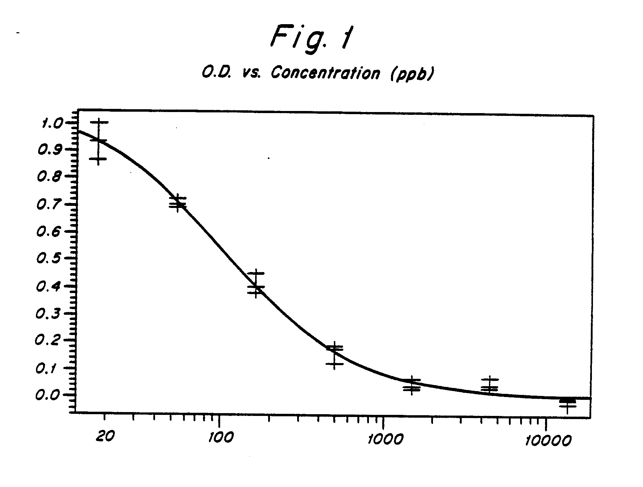

The results of this procedure shown in Fig 1 show a competitive inhibition enzyme immunoassay (CIEIA), which demonstrates the binding of the monoclonal antibodies of this invention with free pentachlorophenol and illustrates the utility of the monoclonal antibodies in the quantitative analysis of pentachlorophenol. The color reaction (O.D. 405 nm) was inversely proportional to the concentration of PCP. Also, the linear portion of the curve extends from greater than 1,000 ppb to below 20 ppb. When this curve was analyzed by the four-parameter log-logit method, the calculated concentration at 90% of the usable O.D. range was 13 ppb, which corresponds to a minimal detectable level. Unknown samples would be run similarly and the resulting O.D. would be compared with the known standard curve. In this way, the monoclonal anti-PCP antibodies can be used to quantify PCP levels. The results compare favorably in sensitivity to the standard gas chromatographic method for PCP analysis. See Federal Register, Vol. 49, No. 209, October 26, 1984.

Table 1 represents the results of tests done to determine the specificity of the antibodies from ATCC HB 9198. Here specificity refers to the cross-reactivity of the anti-PCP antibodies with closely related chemical compounds. The results in Table 1 were expressed as the Molar $IC_{50}$, defined as the molar concentration of a particular chemical that inhibits 50% of the binding of anti-PCP to the coated plate. With the molar $IC_{50}$ of PCP taken as 100%, each compound was compared by a percent cross-reactivity ($IC_{50}PCP/IC_{50}$ compound $\times$ 100). It can be seen that 2,3,5,6-tetrachlorophenol showed 42% cross-reactivity with the antibody from ATCC HB 9198, and 2,4,6-trichlorophenol and 2,3,6-trichlorophenol showed 12% and 8%, respectively. From these data, it is apparent that for significant cross-reactivity with the anti-PCP antibody, a compound must be a phenol with chlorines in the 2 and 6 positions. Note that pentachlorobenzene, which differs from pentachlorophenol by a single atom, fails to react with the antipentachlorophenol antibody.

0 260 829

## TABLE 1
### Specificity of PCP Monoclonal Antibodies

| Compound | Molar $IC_{50}$ | Percent Cross Reactivity With PCP |
|---|---|---|
| Pentachlorophenol | $2.2 \ (\pm 0.3) \times 10^{-6}$ | --- |
| 2,3,5,6-Tetrachlorophenol | $5.3 \ (\pm 0.6) \times 10^{-6}$ | 42.0 |
| Tetrachlorohydroquinone | $2.8 \ (\pm 0.1) \times 10^{-4}$ | 0.8 |
| 2,4,6-Trichlorophenol | $1.8 \ (\pm 0.3) \times 10^{-5}$ | 12.0 |
| 2,3,6-Trichlorophenol | $2.5 \ (\pm 0.1) \times 10^{-5}$ | 8.8 |
| 2,6-Dichlorophenol | $1.2 \ (\pm 0.1) \times 10^{-4}$ | 1.8 |
| 2,3,4-Trichlorophenol | $4.5 \ (\pm 0.3) \times 10^{-4}$ | 0.5 |
| 2,3,5-Trichlorophenol | $4.3 \ (\pm 0.3) \times 10^{-4}$ | 0.5 |
| 2,4-Dichlorophenol | NI | 0 |
| 2,5-Dichlorophenol | NI | 0 |
| 3,5-Dichlorophenol | NI | 0 |
| 3,4-Dichlorophenol | NI | 0 |
| 2,3-Dichlorophenol | NI | 0 |
| 4-Chlorophenol | NI | 0 |
| Phenol | NI | 0 |
| Pentachloroaniline | NI | 0 |
| Pentachlorobenzene | NI | 0 |
| 2,3-Dinitrotoluene | NI | 0 |
| 2,4-Dinitrotoluene | NI | 0 |
| 2,4,5-Trichloronitrobenzene | NI | 0 |

NI = Not Inhibitory

## Claims

1. A method of producing monoclonal antibodies reactive with a chlorinated phenol characterized by producing an immunogenic conjugate of said chlorinated phenol and a macromolecule carrier; immunizing an animal with said conjugate; obtaining antibody-producing cells from said animal; fusing said cells with tumor cells to produce hybridomas; selecting from among said hybridomas at least one hybridoma that produces antibodies reactive with said chlorinated phenol; and recovering said produced antibodies from said selected hybridoma.

2. A method according to claim 1, characterized in that the chlorinated phenol is pentachlorophenol.

3. A method according to claim 1 or 2, characterized in that the immunized animal is a rodent.

4. A method of claim 1, 2 or 3, characterized in that the tumor cells are mouse plasmacytoma or rat myeloma cells.

5. A method according to any of claims 1 to 4, characterized in that the hybridomas have the characteristics of ATCC HB 9197 or ATCC HB 9198 or mutants or variants thereof.

6. A method of producing monoclonal antibodies reactive with pentachlorophenol characterized by producing an immunogenic conjugate of said pentachlorophenol and a protein; immunizing a mouse with said conjugate; recovering antibody-producing cells from the spleen of said mouse; fusing said antibody-producing cells with mouse plasmacytoma or rat myeloma cells deficient in the enzyme hypoxanthine-guanine phosphoribosyl transferase to form hybridomas; selecting at least one of said hybridomas by growth in a medium comprising hypoxanthine, aminopterin, and thymidine; identifying at least one of said

12

hybridomas that produces an antibody to said pentachlorophenol in free or unconjugated form; culturing said identified hybridoma to produce said antibody in a recoverable quantity; and recovering the antibodies produced by said cultured hybridoma.

7. A method of producing monoclonal antibodies to pentachlorophenol characterized by culturing a hybridoma having the characteristics of ATCC HB 9197 or ATCC HB 9198 or mutants or variants thereof in a culture medium and recovering said antibodies from said medium.

8. An in vivo method of producing monoclonal antibodies reactive with a chlorinated phenol characterized by intraperitoneally placing in a histocompatible or immunosuppressed host a hybridoma that produces said antibodies; and recovering the produced antibodies from the ascites fluid of said host.

9. A method according to claim 8, characterized in that the chlorinated phenol is pentachlorophenol.

10. A method according to claim 8 or 9, characterized in that the host is a rodent.

11. A method according to claim 8, 9 or 10, characterized in that the hybridoma has the characteristics of ATCC HB 9197 or ATCC HB 9198 or mutants or variants thereof.

12. A method for producing a continuous cell line that produces monoclonal antibodies to a chlorinated phenol which comprises: producing an immunogenic conjugate of said chlorinated phenol and a macromolecule carrier; immunizing an animal with said conjugate; obtaining antibody-producing cells from said animal; fusing said antibody-producing cells with tumor cells to produce hybridomas; selecting from among said hybridomas a hybridoma that produces antibodies reactive with said chlorinated phenol; and clonally expanding said selected hybridoma into a continuous cell line.

13. A method according to claim 12, characterized in that the chlorinated phenol is pentachlorophenol.

14. A hybridoma which produces a monoclonal antibody characterized in that said antibody is reactive with a chlorinated phenol.

15. A hybridoma according to claim 14, characterized in that the chlorinated phenol is pentachlorophenol.

16. A hybridoma according to claim 14 or 15, characterized in that said hybridoma has the characteristics of ATCC HB 9197 or ATCC HB 9198 or mutants or variants thereof.

17. A monoclonal or polyclonal antibody characterized in that said antibody is reactive with a chlorinated phenol.

18. An antibody according to claim 17, characterized in that the chlorinated phenol is pentachlorophenol.

19. A monoclonal antibody characterized in that said antibody is recovered from the cell line produced by the method of claim 12 or 13.

20. An antibody according to claim 17 or 18, characterized in that the monoclonal antibody is recovered from a hybridoma.

21. An antibody according to claim 20, characterized in that the hybridoma has the characteristics of ATCC HB 9197 or ATCC HB 9198 or mutants or variants thereof.

22. A method for determining the presence or concentration of a chlorinated phenol in a sample by employing an immunoassay, characterized by using a monoclonal antibody reactive with said chlorinated phenol as a reagent in said immunoassay.

23. A method according to claim 22, characterized in that the monoclonal antibody is reactive with pentachlorophenol.

24. A method for detecting the presence or concentration of a chlorinated phenol in a sample characterized by adding monoclonal antibodies reactive with said chlorinated phenol to said sample; and determining the presence or concentration of said chlorinated phenol by an immunoassay in which said monoclonal antibodies are used as a reagent.

25. An immunological procedure for the isolation or removal of a chlorinated phenol from a mixture on the basis of a selective immunological reaction with an antibody specific for said chlorinated phenol, characterized by using a monoclonal antibody.

26. A method for the purification of a chlorinated phenol characterized by contacting material containing said chlorinated phenol with immobilized monoclonal antibodies reactive with said chlorinated phenol to separate the latter from said material by forming immobilized complexes of said antibody and said chlorinated phenol; and separating said chlorinated phenol from said immobilized antibodies to recover said chlorinated phenol in purified form.

27. A method for the biochemical, immunological, functional, or other investigational analysis of a chlorinated phenol, characterized by using a monoclonal antibody reactive with said chlorinated phenol and in an amount effective to result in said analysis.

28. A composition for determining the presence or concentration of a chlorinated phenol in a sample characterized in that said composition is an effective amount of a monoclonal antibody reactive with said chlorinated phenol and in an acceptable carrier.

29. A composition according to claim 28, characterized in that the chlorinated phenol is pentachlorophenol.

30. A composition for isolating or removing a chlorinated phenol from a mixture containing said chlorinated phenol characterized in that said composition is an effective amount of a monoclonal antibody reactive with said chlorinated phenol and immobilized on an acceptable matrix or in admixture with an acceptable carrier.

31. A composition for the purification of a chlorinated phenol characterized in that said composition is a monoclonal antibody reactive with said chlorinated phenol and immobilized on an acceptable support or carrier.

32. A composition for the biochemical, immunological, functional, or other investigational analysis of a chlorinated phenol characterized in that said composition is an effective amount of a monoclonal antibody reactive with said chlorinated phenol and in an acceptable carrier.

33. A method of producing polyclonal antibodies reactive with a chlorinated phenol characterized by producing an immunogenic conjugate of said chlorinated phenol and a macromolecule carrier; immunizing an animal with said conjugate; removing blood from said animal; separating the serum from said blood; and recovering the antibodies from said serum.

34. A method according to claim 33, characterized in that the chlorinated phenol is pentachlorophenol.

35. A method for determining the presence or concentration of a chlorinated phenol in a sample by employing an immunoassay, characterized by using a polyclonal antibody reactive with said chlorinated phenol as a reagent in said immunoassay.

36. A method of preparing an immunogenic conjugate of a chlorinated phenol and a macromolecule carrier characterized by covalently attaching a chemical linker to said macromolecule carrier; and covalently attaching a derivative of said chlorinated phenol, said derivative containing a reactive group, to said chemical linker, thereby forming said immunogenic conjugate.

37. A method according to claim 36, characterized in that the chlorinated phenol is pentachlorophenol.

38. A synthetic antigen characterized in that said composition is a chlorinated phenol covalently bonded to a macromolecule carrier.

*Fig. 1*

*O.D. vs. Concentration (ppb)*